# EUROPEAN PATENT SPECIFICATION

(11) **EP 1 694 617 B1**
(45) Date of publication and mention of the grant of the patent: **20.07.2011**
(21) Application number: 04804075.2
(22) Date of filing: 16.12.2004
(51) Int. Cl.: C07C 2/18, C07C 2/12, C07C 2/70, C07C 2/66

(54) **IMPROVEMENTS IN OR RELATING TO CATALYSED REACTIONS**
VERBESSERUNGEN IN ODER IN BEZUG AUF KATALYTISIERTEN REAKTIONEN
PERFECTIONNEMENTS RELATIFS A DES REACTIONS CATALYSEES

(30) Priority: 18.12.2003 US 530777 P
(43) Date of publication of application: 30.08.2006
(73) Proprietor: ExxonMobil Chemical Patents Inc., Baytown, TX 77520-5200 (US)
(72) Inventor: BEADLE, Stephen, W., Prairieville, LA 70769 (US); BROWN, Stephen, H., B-1200 Brussels (BE); GODSMARK, John, S., B-1390 Grez Doiceau (BE); MATHYS, Georges, M., K., B-3360 Bierbeek (BE)
(74) Representative: Wall, Leythem
(86) International application number: PCT/EP2004/014475
(87) International publication number: WO 2005/058777

(56) References cited:
- US-A- 2 681 374
- US-A- 3 864 346
- US-A- 4 018 846
- US-A- 5 672 800
- CAVANI F ET AL: "EFFECT OF WATER IN THE PERFORMANCE OF THE SOLID PHOSPHORIC ACID CATALYST FOR ALKYLATION OF BENZENE TO CUMENE AND FOR OLIGOMERIZATION OF PROPENE" APPLIED CATALYSIS A: GENERAL, ELSEVIER SCIENCE, AMSTERDAM, NL, vol. 97, no. 2, 23 April 1993 (1993-04-23), pages 177-196, XP001034799 ISSN: 0926-860X cited in the application

## Description

The present invention relates to improvements in or relating to acid catalysed petrochemical reactions, in particular to the oligomerisation of olefins and alkylation with olefins.

The condensation reaction of an olefin or a mixture of olefins over an acid catalyst to form higher molecular weight products is a widely used commercial process. This type of condensation reaction is referred to herein as an oligomerisation reaction, and the products are low molecular weight oligomers which are formed by the condensation of up to 12, typically 2, 3 or 4, but up to 5, 6, 7, or even 8 olefin molecules with each other. As used herein, the term 'oligomerisation' is used to refer to a process for the formation of oligomers and/or polymers. Low molecular weight olefins (such as ethylene, propene, 2-methylpropene, 1-butene and 2-butenes, pentenes and hexenes) can be converted by oligomerisation over a solid phosphoric acid catalyst, an acidic ion-exchange resin, or a zeolite catalyst, to a product which is comprised of oligomers and which is of value as a high-octane gasoline blending stock and as a starting material for the production of chemical intermediates and end-products. Such chemical intermediates and end-products include alcohols, detergents and esters such as plasticiser esters and synthetic lubricants. The reactions typically take place in a plurality of tubular or chamber reactors. Sulfated zirconia, liquid phosphoric acid and sulfuric acid are also known catalysts for oligomerisation.

The acid catalysed alkylation of aromatic and phenolic compounds with olefins is a well-known reaction which is also of commercial importance. For example, ethylbenzene, cumene and detergent alkylate are produced by the alkylations of benzene with ethylene, propene and C₈ to C₁₈ olefins, respectively. Sulphuric acid, HF, phosphoric acid, aluminium chloride, and optionally supported boron fluoride are conventional catalysts for this reaction. In addition, solid acids which have comparable acid strength can also be utilised to catalyse this process, and such materials include amorphous and crystalline aluminosilicates, clays, ion-exchange resins, mixed oxides and supported acids such as solid phosphoric acid catalysts. It is known that many of these acid catalysts require the presence of a certain amount of water in order to provide optimal catalyst activity.

Solid phosphoric acid catalysts are typically prepared by combining a phosphoric acid with a support and drying the resulting material. A commonly used catalyst is prepared by mixing kieselguhr with phosphoric acid, extruding the resulting paste, and calcining the extruded material. The activity of a solid phosphoric acid catalyst is related to the amount and the chemical composition of the phosphoric acid which is deposited on the support.

Phosphoric acid consists of a family of acids, which exist in equilibrium with each other and differ from each other in their degree of condensation. The catalysts are generally supported on silica and consist of silicone phosphate crystals coated with various phosphoric acids. These acids include ortho-phosphoric acid (H₃PO₄), pyro-phosphoric acid (H₄P₂O₇), triphosphoric acid (H₃P₃O₁₀), and polyphosphoric acids, and the precise composition of a given sample of phosphoric acid will be a function of the P₂O₅ and water content of the sample. As the water content of the acid decreases the degree of condensation of the acid increases. Each of the various phosphoric acids has a unique acid strength and accordingly the catalytic activity of a given sample of solid phosphoric acid catalyst will depend on the P₂O₅/H₂O ratio of the phosphoric acid which is deposited on the surface of the crystals.

The activity of a solid phosphoric acid catalyst and also its rate of deactivation in a hydrocarbon conversion process, such as an oligomerisation or an alkylation process, will be a function of the degree of catalyst hydration. In an olefin oligomerisation process, a properly hydrated solid phosphoric acid catalyst can be used to convert over 95% of the olefins in a feedstock to higher molecular weight oligomers. However, if the catalyst contains too little water, it tends to have a very high acidity, which can lead to rapid deactivation as a consequence of coking. Further hydration of the catalyst serves to reduce its acidity and reduces its tendency toward rapid deactivation through coke formation. On the other hand, excessive hydration of a solid phosphoric acid catalyst can cause a change in the crystal structure, leading to lower density and swelling. This change may cause the catalyst to soften and physically agglomerate and, as a consequence, can create high pressure drops in fixed bed reactors. Accordingly, there is an optimum level of hydration for a solid phosphoric acid catalyst.

During use as a catalyst for hydrocarbon conversion processes, a solid phosphoric acid catalyst will develop a degree of hydration which is a function of feedstock composition and reaction conditions. For example the level of hydration is affected by the water content of the feedstock which is being contacted with the catalyst and also by the temperature and pressure at which the catalyst is used. The vapour pressure of water over a solid phosphoric acid catalyst varies with temperature and it is important to keep the water content of the feedstock in equilibrium with that of the catalyst it is being contacted with. If a substantially anhydrous hydrocarbon feedstock is used with a properly hydrated catalyst, the catalyst will typically lose water during use, and will develop a less than optimal degree of hydration. Accordingly when the water content of a feedstock is inadequate to maintain an optimal level of catalyst hydration, it has been conventional to inject additional water into the feedstock. A study of the effect of water on the performance of solid phosphoric acid catalysts as catalysts for the alkylation of benzene with propene and for the oligomerisation of propene is set forth in a review article by Cavani et al, Applied Catalysis A: General, 97, pp. 177-1196 (1993).

As an alternative to incorporating water into a feedstock that is being contacted with a solid phosphoric acid catalyst, it has also been proposed to add a small amount of an alcohol, such as 2-propanol, to the feedstock, to maintain the catalyst at a satisfactory level of hydration. For example, US Patent No. 4,334,118 discloses that in the polymerisation of C₃-C₁₂ olefins over a solid phosphoric acid catalyst which has a siliceous support, the catalyst activity can be maintained at a desirable level by including a minor amount of an alkanol in the olefin feedstock. It is stated that the alcohol undergoes dehydration upon contact with the catalyst, and that the resulting water then acts to maintain the catalyst hydration.

US 3,864,346 discloses a process in which an olefin is reacted with an isoparaffin in the presence of a sulphuric acid or hydrofluoric acid catalyst. A control system for keeping the acid at the desired strength includes a water analyzer for monitoring the water content of the olefin and isoparaffin. US 4,018,846 also discloses a process in which the water content of an alkylation catalyst comprising water and sulphuric or hydrofluoric acid is monitored.

US 2,681,374 discloses a method for maintaining the strength of a liquid phosphoric acid catalyst in an olefin polymerisation reactor, in which the water content of a wet olefinic feed is monitored by a water analyzer.

As well as using solid phosphoric acid catalysts, it is known to use acidic zeolite catalysts for the oligomerisation of olefins. PCT publication WO 93/16020 discloses the use of such zeolites, and also that the selectivity and the conversion of the oligomeristion process can be improved by the addition of small amounts of water to the oligomerisation reaction.

Where a zeolite catalyst is used it may be any zeolite that is active in alkene oligomerisation reactions. For example, there may be used a catalyst selected from the group consisting of zeolites of the TON structure type (for example, H-ZSM-22, H-ISI-1, H-Theta-1, H-Nu-10, KZ-2), or zeolites of the MTT structure type (for example H-ZSM-23, KZ-1) or zeolites of the MFI structure type (for example, H-ZSM-5) or zeolites of the MEL structure type (for example, H-ZSM-11) or zeolites of the MTW structure type (for example, H-ZSM-12), or zeolites with the EUO structure type (for example, EU-1), or zeolite H-ZSM-57, or any member of the ferrierite structure family. Other examples of suitable catalysts are offretites, H-ZSM-4, H-ZSM-18 or zeolite Beta. Reference is made to 'Synthesis of High-Silica Aluminosilicate Zeolites' by P. A. Jacobs and J. A. Martens (published as volume 33 in the series 'Studies in Surface Science and Catalysis') for a review of the synthesis and properties of the aforementioned zeolites.

Additionally, the catalyst can be a zeolite synthesised without addition of a template, for example, faujasites, zeolite L, mordenites, erioites and chabazites, the structures of which are contained in the 'Atlas of Zeolite Structure Types' by C. Baerlocher, W. M. Meier and D. H. Olson (published by Elsevier on behalf of the Structure Commission of the International Zeolite Association, 5th Revised Edition, 2001). Zeolite catalysts having crystal structures that are essentially the same as the crystal structures of the above-mentioned zeolite catalysts, but differing slightly therefrom in chemical composition, may also be used. Examples include zeolite catalysts obtained by removal of a number of aluminium ions from, or by steaming of, the above-mentioned zeolite catalysts; and zeolite catalysts obtained by the addition of different elements (for example boron, iron and gallium), for example, by impregnation or cation exchange, or by incorporation during the zeolite synthesis.

Mixtures of two or more zeolites e.g. a mixture of ZSM-22 and ZSM-57 or ZSM-22 and ZSM-5 can be used as disclosed in EP 0 746,538B1. Or alternatively, upon the surface zeolite of each crystal, a layer of another zeolite can be deposited as disclosed in EP 0808298B1.

The zeolite conveniently has a crystallite size up to 5 µm such as within the range of from 0.05 to 5µm, for example from 0.05 to 2.0µm, and typically from 0.1 to 1µm. An as-synthesized zeolite is advantageously converted to its acid form, for example by acid treatment, e.g. by HCl, or by ammonium ion exchange, and subsequently calcined before use in the process of invention. The calcined materials may be post- treated, such as by steaming. It is also possible to use, as is known in the art, a material in which silicon and aluminium have been replaced in whole or in part by other elements. Silicon may, for example, be replaced by germanium and/or phosphorus; and aluminium more especially by boron, galium, chromium and iron. Materials containing such replacement lattice elements are also generally termed zeolites, and the term is used in this broader sense in this specification. The zeolite might be supported or unsupported, for example in the powder form, or used as an extrudate with an appropiate binder. Where a binder is employed, the binder is conveniently a metal oxide, such as alumina or silica and is present in an amount such that the oligomerisation catalyst contains for example from 1 to 99wt% of the zeolite, more preferably from 50 to 70wt%.

It is also known from, for example, United States Patents 4334118 and 5744679 that by using in an alkene oligomerisation process an alkene-containing feedstock with a water content of from 0.05 to 0.25 mol %, and preferably of at least 0.06 mol %, based on the hydrocarbon content of the feedstock, the yields of the desired higher molecular weight alkenes can be increased, and the catalyst becomes deactivated more slowly. This is known for both solid phosphoric acid catalysed oligomerisation and zeolite catalysed oligomerisation.

It is also known that when an alkene-containing feedstock has a water content of less than 0.05 mol %, the water content may be increased by a variety of means. For example, the feedstock can be passed through a thermostatic water saturator. Since the amount of water required to saturate the alkene feedstock depends upon the temperature of the feedstock, control of the water content can then be affected by appropriate control of the temperature of the feedstock. The water content of the feedstock is preferably at least 0.06 mol %, based on the hydrocarbon content of the feedstock.

The present invention may be used with particular advantage in the oligomerisation of C₃-C₆-alkenes. If, as may be desired, the alkene-containing feedstock contains as diluent a hydrocarbon other than a C₂-C₁₂-alkene, for example, a saturated hydrocarbon, that other hydrocarbon is to be included in the hydrocarbon content for the purposes of calculation of the water content.

Accordingly with the use of either phosphoric acid or zeolite catalyst it is known that water may be added to enhance catalyst life and possibly also improve selectivity, and to moderate conversion and spread it out over a larger part of the catalyst bed. Oligomerisation reactions typically run over many weeks or months before a catalyst change is required and it is desirable to optimise the selectivity and conversion of the reaction throughout the run. United States Patent 6,111,159 indicates the difficulties that can occur during the initiation of a reaction run and presents a somewhat complicated and expensive solution employing a nonreactive water free hydrocarbon diluent over an extended initial period. The implication in the use of such technology is that the reactor spends several days on line with low catalyst activity and low olefin conversion before optimum reaction conditions are achieved.

Industrial hydrocarbon conversion processes employing these acidic catalysts typically run for several weeks before a catalyst change is required or a decomissioning of the reactor is needed. In industrial processes the feeds for the reactions are generally obtained from refining activities such as a stream derived from catalytic or steam cracking, which may have been subjected to fractionation. The nature of such refining activities is such that there will be variations in the composition of the feed. In addition it may be desired to change the nature of the feed during a reactor run. The catalyst activity and the reaction conditions vary according to the composition of the feed. Furthermore, the reactions are exothermic and the size of the exotherm also depends upon the composition of the feed.

The present invention is concerned with the optimisation of the reaction conditions by adjustments of the amount of water present in the feed.

In most industrial processes such as that described previously, the refinery feed that is to be used in the hydrocarbon conversion reactions will contain impurities such as polar compounds. These impurities would be detrimental to the hydrocarbon conversion reaction and are frequently removed prior to the reaction, by for instance a water wash. In olefin oligomerisation the feeds are frequently subject to a first alkaline wash to remove acidic polar species followed by a weakly acidic water wash. The last water wash typically produces a feed stream which is saturated with water at the temperature at which the water wash is performed and, accordingly, can be used to provide the water of hydration required in the reaction.

The activity of the catalyst in relation to the particular feed that is being processed is controlled by the amount of water present. The requirement for the presence of water varies according to the nature of the catalyst. For example, when using a solid phosphoric acid catalyst water is required for catalyst activation; whereas when using a zeolite catalyst water may be required to temper catalyst activity. Thus the presence of water and the amount of water present is important in all these circumstances.

In hydrocarbon conversion reactors there is generally a temperature profile throughout the reactor, the optimum profile depending upon the nature of the feed and the catalyst. We have now found that particular optimum reactor conditions may be sustained throughout a reactor run if the water content of the feed is continuously controlled according to the composition of the feed.

The present invention provides a process in which higher activity and conversion may be realised earlier in the reactor run and/or in which the activity and conversion can be maintained during the run despite fluctuations and/or changes in the composition of the reaction feed.

According to the present invention there is provided a process for the conversion of olefins in which the conversion reaction comprises the oligomerisation of olefins or the alkylation of aromatic or phenolic compounds with olefins in a reactor, which comprises continuously passing a feed comprising an olefin and water through a bed of catalyst under conversion conditions to form a conversion product, the water content of the feed being automatically controlled according to an analysis of the composition of the reaction feed, and in which the catalyst comprises a zeolite catalyst.

In one embodiment, the present invention provides a process for the conversion of olefins in a reactor which comprises continuously passing a feed comprising an olefin and water through a bed of catalyst under conversion conditions to form a conversion product, the water content of the feed being greater during the initial phase of the process than at the latter phase of the process. In preferred embodiments, this process is practised with one or more process features as expressed elsewhere herein.

The invention therefore includes processes for the oligomerisation of olefins or the alkylation of aromatic compounds with olefins wherein a hydrated olefin feed is continuously passed through a bed of oligomerisation or alkylation catalyst under oligomerisation or alkylation conditions, wherein the water content of the feed is automatically controlled according to the composition of the feed and/or is greater during the initial phase of the process than at the latter phase of the process.

The catalysts are generally acidic and the use of the techniques of this invention allows the optimum acidity of the catalyst to be maintained through the reactor and throughout the reactor run. The preferred oligomerisation or alkylation conditions to be employed will depend upon the particular catalyst that is used, and will generally be such as to provide a desired temperature profile throughout the reactor.

The catalyst comprises a zeolite. The temperature of the conversion, e.g. oligomerisation or alkylation, may be, for example, in the range 110°C to 310°C.

The present automatic process control invention allows optimum conditions to be sustained despite fluctuations and/or changes in the composition of the feed by monitoring the composition of the feed, and adjusting its water content according to the fluctuations or changes identified. In this way it is possible to match a preset target of the free acidity level of the catalyst at average reactor and/or run conditions. In a process in which the olefin feed is subjected to a water wash, it is preferred to adjust the water content by altering the temperature of the water wash to a temperature at which saturation of the feed provides the desired water level.

The difference between "reactor" or "run" conditions depends on the control capabilities. If hydration (i.e. feed water content) control of an individual reactor is possible, the control targets average reactor conditions and adjusts through the run. If only one hydration control is provided for a range of reactors in parallel, which almost inevitably are at different stages of run, the target is for average run conditions. A preferred embodiment of the "reactor" conditions target is where one saturation step is complemented with water injection capabilities into the feeds to each individual reactor, to adjust for its particular (average) operating conditions.

The invention is particularly concerned with the production of C₅ to C₂₀ olefins boiling in the range of 30° to 310°C, preferably 30° to 300°C, more preferably 30° to 250°C, from propylene and/or butene and/or amylene feedstocks or their mixtures, though ethylene may be present as well. In particular the invention is concerned with the production of the following olefins.

| **Products** | **Distillation Range (°C) ASTM D1078** | |
|---|---|---|
| | **Initial Boiling Point** | **Dry Point** |
| Pentenes | 30 | |
| Hexenes | 35 | 72 |
| Heptenes | 88 | 97 |
| Octenes | 114 | 126 |
| Nonenes | 135 | 143 |
| Decenes | 155 | 160 |
| Undecenes | 167 | 178 |
| Propylene Tetramers | 175 | 225 |
| Or Dodecenes Tridecenes | 204 | 213 |

In addition to enabling the yields of higher molecular weight alkenes to be increased, the process of the invention enables the oligomerisation reaction to be carried out at relatively low temperatures. When using a zeolite catalyst the contacting is preferably carried out at a temperature in the range 180°C to 310°C.

We have found that the process of the present invention enables a smoother running of oligomerisation reactions employing a zeolite catalyst. In particular the invention leads to a reduction of excessive exotherms at the start of operation of the reactor loaded with fresh or freshly regenerated catalyst, in case of a regenerable catalyst. We have also found that the present invention enables high catalyst activity and olefin conversion to be maintained despite any changes and/or fluctuations in the composition of the feed.

We have found that in certain reactions employing zeolite catalyst the catalyst activity and acidity are too high initially, and that benefits can be achieved using more water in the olefin feed during the initial period of a reaction than in later stages once stable reaction conditions have been achieved. It is believed that this hydration profile over time contributes to a smoother temperature profile, particularly in tubular reactors, and in avoiding temperature excursions in certain parts of the catalyst beds that could cause excessive coking and hence catalyst deactivation. Preferably the initial phase of the run (employing the higher water content) lasts for up to 48 hours, more preferably up to 24 hours.

Accordingly, where the present invention is applied to olefin oligomerisation, the present invention further provides techniques whereby the water content of the feed may be managed throughout a reaction run in order to optimise the catalyst activity and the olefin conversion. In most oligomerisation processes, the olefin feed to oligomerisation is subjected to a water wash. At this stage the feed may be saturated with water (at the temperature employed in the washing) and the water content may be controlled automatically to the level required by the selected oligomerisation conditions. A convenient technique for the adjustment of the water content of the feed, is to alter the temperature employed in the water wash step. An increase in the temperature will result in a higher water content at saturation, whereas if the temperature is reduced the water content at saturation will be lower. If the water level is too low, more water may be injected into the feed or, in case of several reactors operated in parallel, water may be injected directly into the feed to an individual reactor to achieve the desired level. This control in hydration enables the system to compensate for changes in feed composition, changes in reactor temperature, changes in recycle rates and variations in the proportions of reactive materials (such as olefins) and of diluents (if any are used).

In a preferred embodiment one or more coalescers are provided downstream of the water washing of the feed. These coalescers remove fine water droplets which can lead to excess of water on the catalyst, which degrades the catalyst. Coalescers are devices that are employed to facilitate the separation of two liquid phases. A problem with separating two liquid phases can be that the density difference, which drives the separation, is often rather small. In this situation the smaller droplets travel slowly through the continuous phase (according to Stokes' Law) and in an empty vessel, they have to travel all the way to the bottom (or the top) before they start agglomerating to form larger droplets and ultimately a separate continuous phase. In a coalescer, horizontal (or substantially horizontal) baffles are provided within the vessel in order to reduce the distance the droplets must travel, and thus make the separation more effective and volume-efficient. A preferred coalescer design comprises a drum with a number of parallel baffles inside, which are horizontal or slightly inclined. Small droplets will only need to travel to the baffle that is just below or above it, where they will form larger droplets (by coalescing with each other) which then will travel to the end of the baffle and from there will move with their faster speed to the bottom or top of the vessel where the continuous separate phase is formed and removed through the outlet nozzle. The benefit of using a coalescer in the present invention is that the entrained water content of the organic feed to the oligomerisation reactor will be reduced and hence the water content will be closer to the saturation amount of water. The latter can be estimated by calculation, so the total amount of water in the stream can be more reliably predicted/calculated. This enables a better hydration control by controlling the saturation temperature and/or controlling the amount of water injected into the feed on its way to the oligomerisation reactor. Another embodiment of a coalescer may be one where the fluid containing small droplets of a different phase is pushed through a high porosity solid such as a packing or crinkle-wire-mesh-screen that is made of a material to which the droplet phase has a high affinity or wettability. The small droplets then tend to adhere to this easily wettable material and coagulate to form bigger droplets, which are then released from the material and readily separate into a continuous phase that can be drawn off.

In a further embodiment, an on line analyser is provided to measure the amount of water present in the olefin feed as it is fed to the reactor. Water injection and/or water wash temperature changes or drying systems may then be activated, preferably automatically, according to the analysis to make any adjustments to water content that may be required to optimise the oligomerisation reaction. The analysis may be monitored using one or more on line analysers which take a small slip stream which is analysed by gas chromatography or Panametrics™ analysis technology.

The use of an on line analyser to determine the composition of the hydrocarbon feed to or from the last water wash step, provides a breakdown of the feed's individual hydrocarbon components, typically the individual olefinic and paraffinic components. This composition is then available as the basis for calculating the water solubility of the feed stream at the temperature of the water wash, and may be used to automatically adjust the temperature by an appropriate algorithm. When coalescers are used to assure there is little to no water entrainment, it is preferred that this on line analysis take place after the coalescers, to allow an accurate assessment of the concentration of water in the feed to the reactor. This is of interest because on line water analysers are known for slowly and unnoticeably developing a bias, so one would need to calibrate them regularly. Calibration of these analysers is difficult and troublesome, because analytical standards with low or zero water content are difficult to prepare and to store. The capability to accurately predict the amount of water dissolved in the product of the water wash step is therefore of great benefit for exerting a tight hydration control, either by temperature control in the water wash stage or by injection of additional water, or a combination of both, or by drying.

In yet another further embodiment, the analysis of the reactor feed also includes measuring the concentration of oxygenated components other than water, like alcohols, ethers, or carbonyl containing compounds such as ketones and aldehydes, acids, and the like. Under reactor conditions, these compounds are prone to generating water, which participates in the hydration of the catalyst. Between 40% and 60% of ethers present tend to decompose to generate water, whereas the conversion of most of the alcohols is essentially complete, methanol being the exception. Methanol dehydrates via methylation, which is a much slower reaction, so methanol conversion is significantly lower than its C2 and higher homologues. The additional information on the concentration of the oxygenates in the feed other than water, enables adjustment of the amount of water required for optimal hydration, and hence adjustment of the temperature of the water wash step and/or the amount of water injected and/or the amount of drying.

In yet a further embodiment of the invention, at the end of the run when the catalyst is unloaded from the reactor, the acidity of the spent catalyst recovered closest to the reactor outlet is verified analytically. It is preferred that the acidity of the spent catalyst at the reactor outlet be substantially the same as the acidity level of fresh catalyst. If the acidity of the spent catalyst deviates significantly from the preferred level, the hydration target is adjusted in order to better maintain the target free acidity level for the subsequent runs. The free acidity level of the catalyst may be measured by extracting the free acid from the catalyst, preferably with water, followed by titration. It is preferred that the free acidity (i.e. free phosphoric acid content) of the fresh catalyst and the catalyst at the reactor outlet are both within the range 16 to 22%, more preferably in the range 18 to 20%. Any variation in the acidity of the catalyst at the reactor outlet can be an indicator of the need to adjust the water content in the olefin feed.

In a further embodiment of the invention, the reactor may be provided with means that enable the reactor to be depressurised to flash off water if the water content in the reactor exceeds the desirable level. This can allow the control of water content under circumstances where an undesirable build up of water would, in other circumstances, require the reactor to be decommissioned. It also allows to correct promptly, before excessive damage is done to the catalyst, for a fresh catalyst that was delivered and loaded in overhydrated condition, or when operational upsets have caused the catalyst to become overhydrated. The use of this further embodiment is more applicable to systems that employ tubular reactors than to those that employ chamber reactors. Tubular reactor systems are typically equipped with a vacuum system to evacuate the reactor tubes, and this vacuum can be activated to assist the flashing from the catalyst of water and also hydrocarbons.

In many olefin reaction processes, such as oligomerisation, unreacted olefin is recycled to the reactor and here again the water content and oxygenate content of the olefin recycle feed can be monitored and adjusted to optimise the reaction conditions. Alternatively such recycle is mixed with the fresh feed before the water wash step, and hydration control is effected on the combined stream.

Effective control of the water content of the feed has been found to reduce the undesirable exotherms that can be produced when using acidic zeolite catalysts. It is preferred that the zeolite catalyst may be any catalyst that is active in alkene oligomerisation reactions. For example, there may be used a catalyst selected from the group consisting of zeolites of the TON structure type (for example, H-ZSM-22, H-ISI-1, H-Theta-1, H-Nu-10, KZ-2), or zeolites of the MTT structure type (of example H-ZSM-23, KZ-1) or zeolites of the MFI structure type (for example, H-ZSM-5) or zeolites of the MEL structure type (for example, H-ZSM-11) or zeolites of the MTW structure type (for example, H-ZSM-12), or zeolites with the EUO structure type (for example, EU-1), or zeolite H-ZSM-57, or any member of the ferrierite structure family. Other examples of suitable catalysts are offretites, H-ZSM-4, H-ZSM-18 or zeolite Beta. Reference is made to 'Synthesis of High-Silica Aluminosilicate Zeolites' by P. A. Jacobs and J. A. Martens (published as volume 33 in the series 'Studies in Surface Science and Catalysis') for a review of the synthetics and properties of the aforementioned zeolites.

Additionally, the catalyst can be a zeolite synthesised without addition of a template, for example, faujasites, zeolite L, mordenites, erioites and chabazites, the structures of which are contained in the 'Atlas of Zeolite Structure Types' by W. M. Meier and D. H. Olson (published by Butterworths on behalf of the Structure Commission of the International Zeolite Association). Zeolite catalysts having crystal structures that are essentially the same as the crystal structures of the above-mentioned zeolite catalysts but differ slightly therefrom in chemical composition may also be used, for example, zeolite catalysts obtained by removal of a number of aluminium ions from, or by steaming of, the above-mentioned zeolite catalysts, or zeolite catalysts obtained by addition of different elements, for example, by impregnation or cation exchange or by incorporation during the zeolite synthesis (for example boron, iron and gallium).

The zeolite catalysts or modified zeolite catalysts may be used in the form of powders (including powders consisting wholly or in part of single crystals). The zeolite catalysts may instead be incorporated in shaped agglomerates, for example, tablets, extrudates or spheres, which may be obtained by combining the zeolite with a binder material that is substantially inert under the conditions employed in the oligomerisation process. The zeolite catalyst may be present, for example, in amounts of from 1 to 99% by weight, based on the combined weight of the zeolite and binder material. As binder material there may be used any suitable material, for example, silica, metal oxides, or clays, such as montmorillonite, bentonite and kaolin clays, the clays optionally being calcined or modified chemically prior to use. Further examples of suitable matrix materials include silica-alumina, silica-berylia, silica-magnesia, silica-thoria, silica-alumina-zirconia and silica-magnesiazirconia.

In a further aspect of the invention there is provided a process for oligomerising C₂-C₁₂-alkenes comprising contacting a C₂-C₁₂-alkene-containing feedstock with crystals of H-ZSM-22 or with crystals of a modified zeolite catalyst having the crystalline structure of ZSM-22, the crystals having a length to diameter ratio of not less than 3 and a length of not greater than 30 µm, preferably not greater than 10 µm and, more especially, not great than 1 µm.

In an especially preferred process according to the invention, a C₂-C₁₂-alkene-containing feedstock, more especially a feedstock containing C₃-C₆-alkenes, is contacted with H-ZSM-22 or a modified zeolite catalyst having essentially the crystalline structure of ZSM-22, and the water content is controlled according to the present invention between about 0.05 to 0.25 molar %. We have found that this enables good yields of dimeric, trimeric and tetrameric products to be obtained. Further, good catalyst activity is observed at relatively low temperatures, for example, at temperatures of from 150°, typically 180°, to 255°C. We also found that the catalyst becomes deactivated relatively slowly, as compared with oligomerisation processes where the feedstock is relatively dry.

The alkenes that may be oligomerised by the processes of the invention include ethylene, propene, and linear or branched C₄-C₁₂-alkenes, which may be mono-, di- or polyunsaturated, although di-unsaturated and particularly polyunsaturated compounds are less preferred. As indicated above, however, the alkenes are preferably C₃-C₆-alkenes. The process is particularly advantageous for the oligomerisation of propene and butenes and may be used for the oligomerisation of a single alkene, or of mixtures of alkenes of the same or of different carbon numbers. The alkene may if desired be diluted, for example, with a low molecular weight saturated hydrocarbon, and is preferably mono-unsaturated.

The reaction is preferably carried out at pressures higher than atmospheric pressure, for example, at pressures up to 100 bar (10⁷ Pa).

This invention can be used in the practice of any conventional hydrocarbon conversion process which is carried out over an acid catalyst and in particular a solid phosphoric acid catalyst. Such processes include but are not limited to, the oligomerisation of olefins and the alkylation of aromatics with olefins. Specific examples of such processes include the oligomerisation of C₃ and C₄ olefins to liquids which are useful as gasoline, kerosene or distillate blending stocks; and the preparation of a C₆-C₁₅ olefinic mixture which is useful as a chemical feedstock for the production of isoparaffinic solvents, alcohols, aldehydes and acids. They further include the preparation of ethylbenzene by alkylation of benzene with ethylene, the synthesis of cumene and/or p-diisopropylbenzene by alkylation of benzene or cumene with propene, and the preparation of alkylbenzene or alkylphenols by alkylation of benzene or phenol with C6-C20 olefins.

The use of a start-up fluid is not essential. Particularly in tubular reactors, which provide the capability to heat up the catalyst bed via the heat transfer medium on the shell side of the reactor, this step can be dispensed with. The bed may then be heated by providing a heated medium around the tubes containing the catalyst, and when the desired temperature is reached, normal feed may be introduced into the reactor.

The start-up feed comprises an olefin, optionally a diluent, and the appropriate amount of water. The relative proportions of the materials in this feed depend upon the nature of the olefin and the oligomerisation conditions. The reactions are strongly exothermic and accordingly a diluent such as a paraffinic or a heavy olefinic hydrocarbon is generally used. For example when the feed consists of C₃ olefins and the catalyst is solid phosphoric acid, we prefer (especially when a tubular reactor is used) that the feed contain from 30 % to 60%, more preferably 40 % to 50 %, most preferably 48 to 52% by weight of olefins, with the balance being a paraffinic or a heavy olefinic hydrocarbon diluent, such as a C₃-C₅ refinery paraffinic stream. When employing chamber reactors, we prefer to use feeds with lower olefin strengths, such as 20 to 40, preferably 25 to 35 % by weight. Such feeds may be readily available as that which may be obtained from a catalytic cracker. Its olefin content may be reduced if needed by recycling of unreacted paraffins or low olefinic streams found elsewhere or recovered from the reactor effluent. If butene is to be oligomerised we prefer to use a feed containing from 50 % to 70 % olefins.

The preferred olefin strength in the feed is determined by the extent to which carbon deposits on the catalyst are formed which in turn depends upon the exotherm of the oligomerisation reaction. There is also a minimum olefin content required in the feed to prevent the reaction from ceasing. This minimum is dependent on the space velocity, the temperature, and the nature of the catalyst in the reactor.

Although water is a highly satisfactory hydrating agent, it is relatively insoluble in typical hydrocarbon feedstocks under ambient conditions of temperature and pressure. Accordingly, it is frequently not convenient to incorporate sufficient water in a hydrocarbon feedstock to produce a desired level of catalyst hydration. However monohydric aliphatic alcohols which contain from 2 to 12 carbon atoms are ordinarily quite soluble in typical hydrocarbon feedstocks. Accordingly these alcohols are very convenient for use as hydrating agents. A highly preferred embodiment of the invention involves the use of a hydrocarbon feedstock wherein the hydrating agent is comprised of a mixture of water and at least one alcohol which is selected from the group consisting of monohydric aliphatic alcohols which contain from 2 to 12 carbon atoms. Such a feedstock is conveniently prepared by adding one or more alcohols to the hydrocarbon components of the feedstock which contain water, and wherein the amount of water is insufficient to provide the desired level of catalyst hydration.

In one aspect the process of this invention can be used in connection with the chemical conversion of any hydrocarbon feedstock which is carried out over a fixed bed of solid phosphoric acid catalyst. Such chemical conversions include, but are not limited to olefin oligomerisation reactions and the alkylation of aromatic compounds with olefinic alkylating agents. Suitable olefins for use in such a process include, but are not limited to cyclic olefins, substituted cyclic olefins, and olefins of Formula I, wherein R₁ is a hydrocarbyl group and each R₂ is independently selected from the group consisting of hydrogen and hydrocarbon groups. Preferably R₁ is an alkyl group and each R₂ is independently selected from the group consisting of hydrogen and alkyl groups.

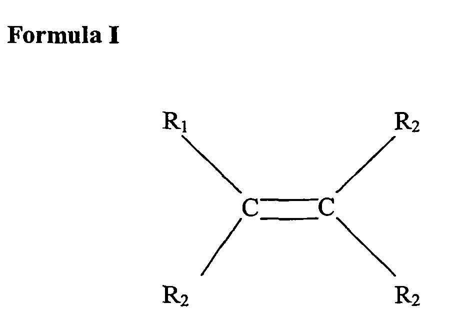

Examples of suitable cyclic olefins and substituted cyclic olefins include, but are not limited to cyclopentene, 1-methylcyclopentene and cyclohexene. Examples of suitable olefins of the type of Formula I include, but are not limited to, propene, 2-methylpropene, 1-butene, 2-butene, 2-methyl-1-butene, 3-methyl-1-butene, 2-methyl-2-butene, 2,3-dimethyl-2-butene, 2-ethyl-1-butene, 1-pentene, 2-pentene, 2-methyl-1-pentene, 3-methyl-1-pentene, 4-methyl-1-pentene, 1-hexene, 2-hexene, and 3-hexene. Suitable olefins will desirably contain from 3 to 12 carbon atoms, preferred olefins will contain from 3 to 6 carbon atoms, and highly preferred olefins will contain from 3 to 4 carbon atoms. Ethylene may also be present, in minor or in major amounts.

The materials obtained from the process of the present invention for the oligomerisation of olefins will generally be a mixture of desired olefin oligomers, unreacted olefins, diluent (if any is used), water and other impurities. The materials are therefore separated, generally by fractional distillation primarily into the olefin oligomers, the unreacted olefins and, if present, the diluent. The unreacted olefins and diluents may be recycled to the oligomerisation reactor. The olefin oligomers may then be purified as required for use in subsequent reactions. For example the oligomers may contain trace amounts of sulphur which may damage a hydroformylation catalyst. Accordingly, if the olefins are to be used as a feed for hydroformylation, the feed may need to be desulphurised. Similarly the olefin oligomers may contain trace amounts of chlorine which may also be detrimental to hydroformylation catalysts and may need to be removed. If the hydroformylation catalyst is not damaged by sulphur or chlorine, the catalyst in the subsequent hydrogenation step to produce the alcohol derivatives may be damaged by these compounds, and hence sulphur and chlorine are preferably removed, most preferably to very low levels. Furthermore the olefin oligomers themselves are frequently mixtures of oligomers of different carbon number. For example oligomerisation of a mixture of propylene, butene and amylene can result in a mixture of C₆ to C₁₃ oligomers and this mixture can then be separated by fractional distillation to obtain the oligomer or oligomer mixtures desired for a particular purpose.

The process of this invention may also be used in connection with the alkylation of an aromatic compound with an olefinic alkylating agent, suitable aromatic compounds include all organic compounds of from 6 to 20 carbon atoms which contain aromatic functionality and can be alkylated by an olefin in the presence of an acidic catalyst such as a solid phosphoric acid catalyst. Such materials include both aromatic compounds and substituted aromatic compounds which carry one or more substituents. Aromatic hydrocarbons and hydrocarbyl substituted aromatic hydrocarbons which contain from 6 to 10 carbon atoms are particular suitable. In addition mixtures of such materials can be used as a substrate. Examples of such material include compounds of Formula II which contain from 6 to 20 carbon atoms where each R is independently selected from the group consisting of hydrogen and hydrocarbyl groups. However preferred aromatic compounds are hydrocarbons which contain from 6 to 10 carbon atoms and are of Formula II where each R is independently selected from the group consisting of hydrogen and alkyl of from 1 to 3 carbon atoms. Benzene and toluene are particularly preferred. 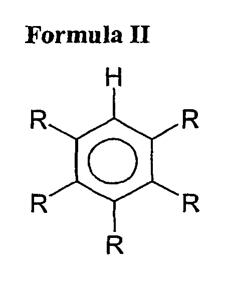 Aromatic compounds for use as alkylation substrates in the practice of this invention can be obtained from any desired source. However, in a petroleum refinery steamcracking units, powerformers, reformers and isomerisation units are convenient sources of the aromatic compounds. For example, a light reformate can be used, and a typical material of this type will have a total aromatic content of about 35 vol % and will contain about 10 vol % of benzene. For processing efficiency, often the higher purity aromatic feeds are preferred due to recycles typically being used in the process.

In a highly preferred embodiment, the process of this invention can be used in connection with the conversion of a mixture of C₃ and C₄ olefins to gasoline blending stock by oligomerisation. In such an embodiment, the feedstock will be comprised of at least about 25 % by volume of olefins. A typical olefin-containing feedstock to a polymerisation unit for conversion to oligomers in the gasoline boiling range will comprise a mixture of propane, butane, 1-methylpropane, propene, 1-butene, 2-butene and 1-methylpropene, wherein the olefin concentration is in the range from about 35 to about 60 vol %. Ethylene and ethane may also be present, albeit typically in minor amounts. However it will be appreciated that the olefin-containing feedstock can have a variety of other compositions which include but are not limited to, other olefins or olefin mixtures, other diluents and the presence of a minor amount of aromatic compounds. In addition olefin concentrations can be used which are outside this range.

In a further embodiment the present invention is used for the oligomerisation of olefins such as ethylene, propylene, butenes and amylenes to produce C₆ to C₁₃ olefins which can be used as feeds for hydroformylation reactions for the production of aldehydes and alcohols. The aldehydes may then be oxidised to produce acids or hydrogenated to produce alcohols. The alcohols may then be used in the production of synthetic esters such as plasticiser esters or synthetic lubricants or in the production of surfactants. The olefins may be hydroformylated using low pressure rhodium catalysed hydroformylation technology or high pressure hydroformylation technology which is typically cobalt catalysed, but rhodium is also used. The present invention is particularly useful in the production of feedstocks which are hydroformylated in the manner described in our copending US patent application 60/530805 filed 18 December 2003 (applicant's reference 2003B137/PM2003-121/HA5)). Where the aldehydes produced by this method are hydrogenated, this may readily be accomplished by the method described in our copending US patent application 60/530804 filed 18 December 2003 (applicant's reference 2003B138/PM2003-120/HA2).

In another highly preferred embodiment the process of this invention can be used in connection with the conversion of a feedstock which comprises low molecular weight aromatic compounds in combination with C₃ and C₄ olefins to produce a product which is in the gasoline boiling point range and useful as a gasoline blending stock. For example, the mol ratio of olefins to aromatic compounds can be in the range from about 1 to about 50, and preferably from about 1 to about 30. In such an embodiment volatile low molecular weight aromatic compounds such as benzene, which are undesirable as gasoline components because of toxicity considerations, can be converted to less volatile materials which are highly desirable gasoline components by alkylation. For example benzene and toluene can be converted to cumene and cymene respectively by monoalkylation with propene.

In those cases where the feedstock contains both olefins and aromatic compounds, alkylation of the aromatic compounds by the olefin or olefins in the feedstock may compete with olefin oligomerisation. As a result, both olefin oligomers and alkylated aromatic compounds will be obtained as products and the ratio of these products will be a function of the mol ratio of olefins to aromatic compounds in the feedstock. For example, when the mol ratio of olefins to aromatic compounds is about 1 to 4, 1 to 5, or even 1 to 8, or 1 to 10 or above the formation of products from alkylation of the aromatic compounds may predominate over the formation of olefin polymerisation products. However when the mol ratio of olefins to aromatic compounds is about 1 or above, the formation of olefin polymers will typically predominate. Such alkylation reactions may be operated in the liquid phase or in the vapour phase, though the vapour phase is typically preferred when using solid phosphoric acid catalyst. When alkylating with ethylene, and the ethylene may be fed in a concentrated or diluted from, even down to 5% in the ethylene feed gas, conditions may be about 300°C and 40-65 bar, and with ethylene to aromatic ratios of 0.1 to 1. With other catalysts, and typically when polyalkylbenzenes are desired, conditions may be 15-20 bar and temperatures of 420-430°C. When alkylating with propylene, typical conditions may be 200-250°C and 15-35 bar. Using zeolites, alkylation may be done at weight hourly space velocities ranging from 1 to 10, in the liquid phase. Involving ethylene, the preferred temperatures are then 180°C to 250°C. Involving propylene, the preferred temperatures are 110°C to 160°C. In both cases, staged injection of the olefin feed may be preferred for better temperature control.

The hydrocarbon conversion process of this invention is preferably carried out in a fixed bed. The reaction can be performed in a chamber or a tubular reactor. In a tubular reactor, the catalyst is contained in a multiplicity of tubes which are surrounded by a circulating cooling medium. These tubes will typically have an internal diameter of from about 5 cm to about 15 cm, although other diameters can also be used. A tubular reactor is frequently preferable to a chamber reactor because it permits a closer control of the reaction temperature and can be easily constructed for high pressure operation. Ordinarily a plurality of reactors will be used. For example an olefin oligomerisation unit employing tubular reactors can have as many as eight or more reactors. The temperature in tubular reactors is typically controlled by steam generation around the reactor tubes. Multiple tube bundles may have their shell side linked up to the same single steam drum.

In a chamber reactor, two or more catalyst beds are incorporated into a single vessel with a diameter in the range, but not exclusively, 1.5 m to 2.5 m. The temperature rise across each bed, due to the reaction, is cooled before the next bed with an external and cooler quench stream. This quench stream is generally predominantly saturated hydrocarbon material and often is the product LPG distilled in the first fractionation tower downstream of the reactors in the process, or vapour condensate from a flash drum located in a similar position. Where a chamber reactor is used the heat produced by the exothermic olefin conversion reactions can be controlled by using a low reactivity hydrocarbon as a recycle from reactor effluent to reactor feedstock and/or as a quench between multiple catalyst beds within the reactor.

Other recycle streams can also be recycled to the reactor to effect dilution or to modify the product slate. For instance, in a propylene fed reactor, C6, C9 or C12 olefin streams, fractionated downstream of the reactors, can be recycled to the reactor to modify the product slate distribution. Byproduct streams of carbon numbers other than the above, such as C7-8 or C10-11 mixtures, can also be recycled to reduce their production, if possible even recycled to their full extinction. The feed to the reactors may also be diluted with such recycle streams.

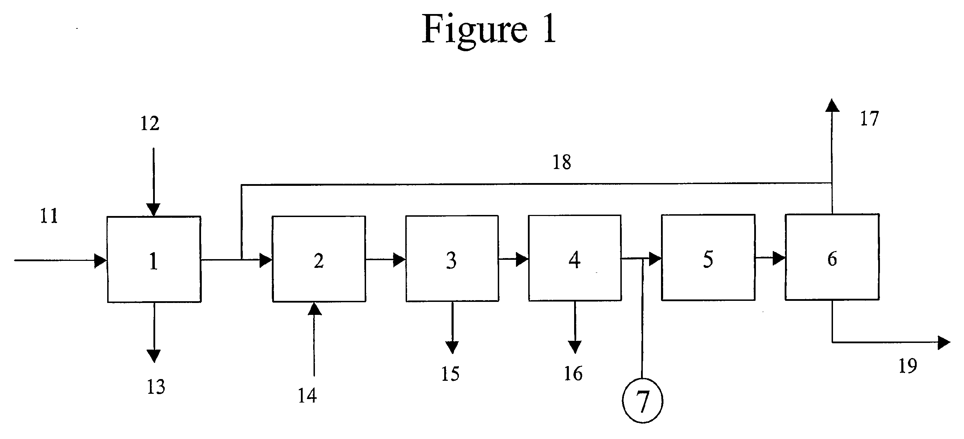

The operation of the present invention in relation to the oligomerisation of olefins is illustrated by the accompanying Figure 1, in which (1) denotes an alkaline wash drum or tower to which olefin feed (11) is fed as is fresh caustic (12). Spent caustic (13) is removed from the alkaline wash operation. The product of the alkaline wash is then typically brought to the desired temperature and sent to a water wash (which may be mildly acidic) at (2), where water (14) is introduced. The olefin stream containing water then passes to a settling drum (3) where water (15) separates out. Part of this water may be recycled to the water wash (2). The olefin stream then passes to a coalescer (4) where also the finer droplets of water (16) are removed and then to the reactor (5). An on line analyser (7) is provided to determine the feed composition and send a signal to alter the hydration level as required, preferably by altering the temperature of the water wash performed at (2). After reaction, the product passes to stabiliser tower (6) where olefin oligomer is separated as bottoms products (19) and the volatiles removed overhead as (17). These volatiles may be partially recycled as shown at (18).

## Claims

1. A process for the conversion of olefins in which the conversion reaction comprises the oligomerisation of olefins or the alkylation of aromatic or phenolic compounds with olefins in a reactor, which comprises continuously passing a feed comprising an olefin and water through a bed of catalyst under conversion conditions to form a conversion product, the water content of the feed being automatically controlled according to an analysis of the composition of the reaction feed, and in which the catalyst comprises a zeolite catalyst.

2. The process according to claim 1 in which the water is introduced into the feed by means of a water wash.

3. The process according to claim 2 in which one or more coalescers are provided downstream of the water wash.

4. The process according to any preceding claim wherein the water content of the feed is automatically controlled in dependence on the results of the analysis by one or more of (a) introducing water into the feed, (b) drying the feed and (c), in the case where a water wash is used, adjusting the temperature of the water wash.

5. The process according to any of the preceding claims wherein an on-line analyser is provided to determine the composition of the feed as it is fed to the reactor.

6. The process according to any of the preceding claims in which the analysis of the reactor feed also includes a measure of the concentration of oxygenated components.

7. The process according to any of the preceding claims wherein the water content of the feed is controlled to be greater during the initial phase of the process than the latter phase of the process.

8. The process according to any of the preceding claims in which the conversion products are separated from unreacted olefins and diluent (if any).

9. The process according to claim 8 in which the unreacted olefins and/or diluent (if any) are recycled to the reactor.

10. The process according to any of the preceding claims in which the conversion is performed in a tubular reactor.

11. The process according to any of claims 1 to 9 in which the conversion is performed in a chamber reactor.

12. The process according to any preceding claim in which the conversion is oligomerisation.

13. The process according to claim 12 wherein the conversion product comprises C5 to C20 olefins in the boiling range of 30°C to 310°C.

14. The process according to claim 12 or 13 which comprises the oligomerisation of a mixture of C3 and C4 olefins.

15. The process according to claim 12 or 13 which comprises the oligomerisation of ethylene, propylene, butenes and/or amylenes to produce C6 to C 15 olefins.

16. The process according to any of claims 12 to 15 in which the conversion products are purified for use in subsequent reactions.

17. The process according to claim 16 in which the conversion products are desulphurised.

18. The process according to any of claims 1 to 11 in which the conversion is alkylation.

19. The process according to claim 1 wherein the temperature of the conversion is from 110°C to 310°C.

## Patentansprüche

1. Verfahren zur Umwandlung von Olefinen, bei dem die Umwandlungsreaktion die Oligomerisierung von Olefinen oder die Alkylierung von aromatischen oder phenolischen Verbindungen mit Olefinen in einem Reaktor umfasst, bei dem ein Einsatzmaterial, das Olefin und Wasser umfasst, kontinuierlich unter Umwandlungsbedingungen durch ein Katalysatorbett geführt wird, um ein Umwandlungsprodukt zu bilden, wobei der Wassergehalt des Einsatzmaterials automatisch gemäß einer Analyse der Zusammensetzung des Reaktionseinsatzmaterials gesteuert wird, und bei dem der Katalysator einen Zeolitkatalysator umfasst.

2. Verfahren nach Anspruch 1, bei dem das Wasser mittels einer Wasserwäsche in das Einsatzmaterial eingebracht wird.

3. Verfahren nach Anspruch 2, bei dem stromabwärts von der Wasserwäsche ein oder mehrere Coalescer bereitgestellt wird oder werden.

4. Verfahren nach einem der vorhergehenden Ansprüche, bei dem der Wassergehalt des Einsatzmaterials in Abhängigkeit von den Ergebnissen der Analyse durch ein oder mehrere von (a) Einbringen von Wasser in das Einsatzmaterial, (b) Trocknen des Einsatzmaterials und (c) in einem Fall, bei dem eine Wasserwäsche verwendet wird, Einstellen der Temperatur der Wasserwäsche, automatisch gesteuert wird.

5. Verfahren nach einem der vorhergehenden Ansprüche, bei dem ein Online-Analysegerät bereitgestellt wird, um die Zusammensetzung des Einsatzmaterials zu bestimmen, während es in den Reaktor eingespeist wird.

6. Verfahren nach einem der vorhergehenden Ansprüche, bei dem die Analyse des Reaktoreinsatzmaterials auch eine Messung der Konzentration von sauerstoffhaltigen Verbindungen einschließt.

7. Verfahren nach einem der vorhergehenden Ansprüche, bei dem der Wassergehalt des Einsatzmaterials so eingestellt wird, dass er während der Anfangsphase des Verfahrens größer ist als in der späteren Phase des Verfahrens.

8. Verfahren nach einem der vorhergehenden Ansprüche, bei dem die Umwandlungsprodukte von umgesetzten Olefinen und Verdünnungsmittel (wenn vorhanden) abgetrennt werden.

9. Verfahren nach Anspruch 8, bei dem die unumgesetzten Olefine und/oder Verdünnungsmittel (wenn vorhanden) in den Reaktor zurückgeführt werden.

10. Verfahren nach einem der vorhergehenden Ansprüche, bei dem die Umwandlung in einem Rohrreaktor durchgeführt wird.

11. Verfahren nach einem der vorhergehenden Ansprüche 1 bis 9, bei dem die Umwandlung in einem Kammerreaktor durchgeführt wird.

12. Verfahren nach einem der vorhergehenden Ansprüche, bei dem die Umwandlung eine Oligomerisierung ist.

13. Verfahren nach Anspruch 12, bei dem das Umwandlungsprodukt C₅- bis C₂₀-Olefine im Siedebereich von 30°C bis 310°C umfasst.

14. Verfahren nach Anspruch 12 oder 13, das die Oligomerisierung einer Mischung von C₃- und C₄-Olefinen umfasst.

15. Verfahren nach Anspruch 12 oder 13, das die Oligomerisierung von Ethylen, Propylen, Butenen und/oder Amylenen umfasst, um C₆- bis C₁₅-Olefine herzustellen.

16. Verfahren nach einem der Ansprüche 12 bis 15, bei dem die Umwandlungsprodukte zur Verwendung in nachfolgenden Reaktionen gereinigt werden.

17. Verfahren nach Anspruch 16, bei dem die Umwandlungsprodukte entschwefelt werden.

18. Verfahren nach einem der Ansprüche 1 bis 11, bei dem die Umwandlung eine Alkylierung ist.

19. Verfahren nach Anspruch 1, bei dem die Temperatur der Umwandlung 110°C bis 310°C beträgt.

## Revendications

1. Procédé de conversion d'oléfines dans lequel la réaction de conversion comprend l'oligomérisation d'oléfines ou l'alkylation de composés aromatiques ou phénoliques avec des oléfines dans un réacteur, qui comprend le fait d'envoyer en continu une charge comprenant une oléfine et de l'eau à travers un lit de catalyseur dans des conditions de conversion pour former un produit de conversion, la teneur en eau de la charge étant ajustée automatiquement selon une analyse de la composition de la charge réactionnelle, et dans lequel le catalyseur comprend un catalyseur zéolithique.

2. Procédé selon la revendication 1 dans lequel l'eau est introduite dans la charge par le biais d'un lavage à l'eau.

3. Procédé selon la revendication 2 dans lequel un ou plusieurs agents de coalescence sont amenés en aval du lavage à l'eau.

4. Procédé selon l'une quelconque des revendications précédentes dans lequel la teneur en eau de la charge est ajustée automatiquement en fonction des résultats de l'analyse par une ou plusieurs des étapes suivantes (a) introduction d'eau dans la charge, (b) séchage de la charge et (c), dans le cas où l'on utilise un lavage à l'eau, ajustement de la température de lavage à l'eau.

5. Procédé selon l'une quelconque des revendications précédentes dans lequel un instrument d'analyse en ligne est prévu pour déterminer la composition de la charge à mesure qu'elle est chargée dans le réacteur.

6. Procédé selon l'une quelconque des revendications précédentes dans lequel l'analyse de la charge du réacteur fait également appel à la mesure de la concentration des constituants oxygénés.

7. Procédé selon l'une quelconque des revendications précédentes dans lequel la teneur en eau de la charge est ajustée de façon à être plus élevée pendant la phase initiale du procédé que pendant la dernière phase du procédé.

8. Procédé selon l'une quelconque des revendications précédentes dans lequel les produits de conversion sont séparés des oléfines n'ayant pas réagi et du diluant (éventuel).

9. Procédé selon la revendication 8 dans lequel les oléfines n'ayant pas réagi et/ou le diluant (éventuel) sont recyclés vers le réacteur.

10. Procédé selon l'une quelconque des revendications précédentes dans lequel la conversion est réalisée dans un réacteur tubulaire.

11. Procédé selon l'une quelconque des revendications 1 à 9 dans lequel la conversion est réalisée dans un réacteur à chambre.

12. Procédé selon l'une quelconque des revendications précédentes dans lequel la conversion est une oligo-mérisation.

13. Procédé selon la revendication 12 dans lequel le produit de conversion comprend des oléfines en C₅ à C₂₀ dans le domaine d'ébullition de 30°C à 310°C.

14. Procédé selon la revendication 12 ou 13 qui comprend l'oligomérisation d'un mélange d'oléfines en C₃ et C₄.

15. Procédé selon la revendication 12 ou 13 qui comprend l'oligomérisation d'éthylène, de propylène, de butènes et/ou d'amylènes pour produire des oléfines en C₆ à C₁₅.

16. Procédé selon l'une quelconque des revendications 12 à 15 dans lequel les produits de conversion sont purifiés pour être utilisés dans des réactions ultérieures.

17. Procédé selon la revendication 16 dans lequel les produits de conversion sont désulfurés.

18. Procédé selon l'une quelconque des revendications 1 à 11 dans lequel la conversion est une alkylation.

19. Procédé selon la revendication 1 dans lequel la température de la conversion est de 110°C à 310°C.
